# EUROPEAN PATENT SPECIFICATION

(11) **EP 1 120 646 B1**
(45) Date of publication and mention of the grant of the patent: **09.12.2009**
(21) Application number: 00948305.8
(22) Date of filing: 01.08.2000
(51) Int. Cl.: G01N 27/416, G01N 33/53, G01N 27/12, C12M 1/00, C12N 15/00, C12Q 1/68

(54) **METHOD FOR DETECTING SINGLE NUCLEOTIDE POLYMORPHISMS (SNP) AND POINT MUTATIONS IN GENES**
VERFAHREN ZUM NACHWEIS EINZELNER NUKLEOTIDPOLYMORPHISMEN (SNP) UND PUNKTMUTATIONEN IN GENEN
PROCEDE PERMETTANT DE DETECTER DES POLYMORPHISMES D'UN SEUL NUCLEOTIDE (SNP) ET DES MUTATIONS PONCTUELLES DANS DES GENES

(30) Priority: 06.08.1999 JP 22468199
(43) Date of publication of application: 01.08.2001
(73) Proprietor: Toppan Printing Co., Ltd., Tokyo 101-8560 (JP); Uchida, Kazuhiko, Tsukuba-shi, Ibaraki 305-0035 (JP); Takenaka, Shigeori, Koga-shi, Fukuoka 811-3114 (JP)
(72) Inventor: Takenaka, Shigeori, Koga-shi, Fukuoka 811-3114 (JP); Uchida, Kazuhiko, Tsukuba-shi, Ibaraki 305-0035 (JP); Miyahara, Takatoshi, Chiba-shi, Chiba 261-0003 (JP)
(74) Representative: Grünecker, Kinkeldey, Stockmair & Schwanhäusser Anwaltssozietät
(86) International application number: PCT/JP2000/005093
(87) International publication number: WO 2001/011351

(56) References cited:
- EP-A- 0 882 981
- EP-A2- 0 667 398
- JP-A- 9 288 080
- JP-A- 10 146 183
- US-A- 5 605 662
- US-A- 5 776 672
- TAKENAKA S ET AL: "Electrochemically active threading intercalator with high double stranded DNA selectivity" CHEMICAL COMMUNICATIONS, 1998, pages 1111-1112, XP002387064
- KELLEY S O ET AL: "Long-Range Electron Transfer through DNA films" ANGEWANDTE CHEMIE. INTERNATIONAL EDITION, WILEY VCH VERLAG, WEINHEIM, DE, vol. 38, no. 7, 1 April 1999 (1999-04-01), pages 941-945, XP002162320 ISSN: 1433-7851
- MILLAN K M ET AL: "Voltammetric DNA Biosensor for Cystic Fibrosis Based on a Modified Carbon Paste Electrode" ANALYTICAL CHEMISTRY, AMERICAN CHEMICAL SOCIETY. COLUMBUS, US, vol. 66, no. 18, 15 September 1994 (1994-09-15), pages 2943-2948, XP000478033 ISSN: 0003-2700
- WANG J: "Towards Genoelectronics: Electrochemical Biosensing of DNA Hybridization" CHEMISTRY - A EUROPEAN JOURNAL, WILEY-VCH, vol. 5, no. 6, 28 May 1999 (1999-05-28), pages 1681-1685, XP002387065 WEINHEIM, GERMANY
- WAKAI JUNKO ET AL: "A novel method of identifying genetic mutations using an electrochemical DNA array" NUCLEIC ACIDS RESEARCH, vol. 32, no. 18, 2004, page e141, XP002387199 ISSN: 1362-4962
- KELLEY S O ET AL: "Single-base mismatch detection based on charge transduction through DNA" NUCLEIC ACIDS RESEARCH, OXFORD UNIVERSITY PRESS, vol. 27, no. 24, 15 December 1999 (1999-12-15), pages 4830-4837, XP002235954 SURREY, GB ISSN: 0305-1048
- PATRICK N. GILLES ET AL.: 'Single nucleotide polymorphic discrimination by an electronic dot blot assay on semiconductor microchips' NATURE BIOTECHNOLOGY vol. 17, no. 4, April 1999, pages 365 - 370, XP002932605

## Description

### Technical Field

The present invention relates to a method of, and a detecting apparatus and a detecting chip for detecting single base substitution SNP and point mutation of genes, capable of detecting and analyzing single base substitution SNP (Single nucleotide polymorphism: a genetic mutant in the human genetic code) and point mutation of genetic DNAS as well as gene expression.

### Background art

Specific base substitution SNP is a specific base variation in a base sequence, and it is estimated that one SNP exists in every 1000bp to 2000bp human DNAS, and hundreds of thousands to millions of SNPs are estimated to exist in a human genome, regardless of its health, and single base substitution SNPs are expected to be valuable markers for identifying and preventing causes of disease.

Point mutation is a single base variation in the base sequence of known genes, that may cause functional abnormality of the corresponding protein and be a factor for disease.

As means for detecting and analyzing the differences among base sequences of genetic DNA, the DNA sequencing method (specific base sequence determination method), the PCR-SSCP (Polymerase chain reaction-single stranded polymorphism) method, the allele-specific hybridization method, the DNA chip method and the like are employed.

The DNA sequencing method has two alternatives, the Maxam Gilbert method and the Sanger (dideoxy) method, and in present, the dideoxy method is mainly employed. After amplifying the area of human genes to be analyzed by the PCR method (polymerase chain reaction method), sequencing is performed by using a primer employed in the PCR method or a primer established within the amplified DNA to determine a gene arrangement within the area. Single base substitution SNP and point mutation are detected by conducting the above operation with different DNA samples.

In the PCR-SSCP method (Polymerase chain reaction-single stranded polymorphism method), after amplifying an area of a human gene to be analyzed by the PCR method, the amplified segment is thermally denaturated to single strands, and then electrophoresis of the single strands is conducted in non-denaturation polyacrylamide gel, so that the secondary structure (intramolecular hydrogen bond) of each strand of double-stranded DNA amplified by the PCR method are kept intact. As the secondary structure (Intramolecular hydrogen bond) to be formed depends on specific base sequence, single base substitution SNP and point mutation of genes are detected by the difference in the distance of electrophoresis.

An Allele-specific hybridization method consists in, after amplifying the area to be analyzed in the PCR method, making PCR products or oligonucleotide probes with approximately 20 bases and imobilizing them in an area of membrane (nylon filter), followed by hybridization of DNA samples (detected DNA) labeled by radioisotope 32P, etc. By adjusting the conditions of hybridization such as temperature at the time of the hybridization, specific base sequence substitution SNP and point mutation of genes are detected by the difference of the intensity of the radioisotope.

In principle, the DNA chip method is nearly the same as the allele-specific hybridization method, and is for arranging PCR products or oligonucleotide probes with approximately 20 bases on the stationary phase (on the board), so that hybridization of fluorescence labeled DNA samples (target DNA) on the stationary phase is conducted. By adjusting hybridization conditions such as temperature, single base substitution SNP and point mutation of human genes are detected depending on the intensity of the fluorescence.

In hybridization by the allele-specific hybridization method, a problem is the high cost for treatment and care of the radioisotope that is used to label DNA. Furthermore, in the DNA chip method, the problems when using the fluorescent dye to label the DNA are: (1) the fluorescence is not taken to the DNA frequently enough due to the large molecular structure of the fluorescent dye, therefore the fluorescent intensity of the flourescence labeled probe is not high, (2) fading of the flourescence and (3) flourescence of the board such as glass (flourescence of background).

In order to solve these problems, a method for electrochemically detecting double-stranded DNA and detecting hybrid organizers by fixing probe DNA to the electrode to make it react with DNA samples under the existence of intercalator has been disclosed (see Patent Laid-Open Publication Hei 9-288080 and report of 57 Analytical Chemistry Discussion series 1996, pp137-138) as an easy and highly-sensitive method for detecting DNA hybridization and double-stranded DNA.

However, as the number of single base substitution SNP of genes or point mutation of genes is immense, for example, at least two million single base substitutions SNP are needed to be identified to prepare single a base substitution SNP map at the density of 15KB (definition) for humans. Furthermore, the number of point mutation of genes related to known diseases is also high. By the conventional methods, it is realistically almost impossible to exhaustively analyze single base substitution and point mutation

United States Patent 5,776,672 is directed to a gene detection method. A gene detection sensor has a nucleic acid probe immobilized on the surface of a carrier sensitive to a physical change. A reaction vessel stores a sample solution containing the gene sample denatured into a single-stranded form. A double-stranded nucleic acid is formed in said reaction vessel on the gene sensor by hybridization of the gene sample and the nucleic acid probe immobilized on the surface of the gene sensor. A detection vessel stores a double-stranded nucleic acid recognizing substance, which reacts with the double-stranded nucleic acid, formed on the surface of the gene sensor.

United States Patent 5,605,662 is directed to active programmable electronic devices for molecular biological analysis and diagnosis. A microelectronic device actively carries out and controls molecular biological reactions in microscopic formats. The device consists of addressable microscopic locations. Each micro-location has a surface for the covalent attachment of specific binding entities, a permeation layer, and an underlying microelectrode. The device electronically directs and controls rapid movement and concentration of analytes and reactants to or from any of its micro-locations.

United States patent 6,126,800 is directed to a micro system with an integrated cuvette for the analysis of liquids. An analysis micro system with integrated cuvette comprising: a chip support having at least one cuvette, at least one chip arranged in each cuvette, the chip comprising analysis electrodes and addressing electrodes, and connection means to connect the addressing electrodes to at least one electrical apparatus external to the micro system.

The present invention is for the purpose of solving the above problems of the conventional methods, and provides an apparatus for identifying single base substitution SNP and point mutation capable of detecting and analyzing a large amount of single base substitution SNP and point mutation, that is, capable of conducting treatment of high-through put (in high speed and volume) as well as detecting and analyzing single base substitutions and point mutation with high-sensitivity for a plurality of DNA samples. In short, the present invention intends to realize a large amount of and high-sensitive detecting and analyzing apparatus for single base substitution SNP and point mutation on the basis of the principle of electrochemically detecting double-stranded DNAs and hybrid organizers described in the Patent Laid-open Publication Hei 9(1997)-288080.

### Disclosure of the Invention

In order to solve the above problems, the present invention provides a method for detecting single base substitution SNP and point mutation of genes using a detecting apparatus as defined in claim 1, comprising a closed internal space part capable of being filled with DNA samples and removing DNA samples, a plurality of gold electrodes corresponding to measuring electrodes formed at the bottom of the space part and common electrode which is a counter electrode to the gold electrodes, being arranged not to contact the gold electrodes in the space part, wherein PCR products or oligonucleotides consisting of different genetic sequences are immobilized on each of the gold electrodes, and wherein voltage is applied between the common electrode and the gold electrodes so that electric current which is created between the common electrode and the gold electrodes can be detected.

Further, the present specification also describes a detecting apparatus for single base substitution SNP and point mutation of genes, comprising a closed space part capable of being filled with DNA samples and removing DNA samples, a plurality of gold electrodes corresponding to measuring electrodes formed at the bottom of the space part, common electrode which is a counter electrode to the gold electrodes, being arranged not to contact the gold electrodes in the space part, and a measuring apparatus capable of detecting electric current through the application of voltage between the common electrode and the gold electrodes, wherein PCR products or oligonucleotides consisting of different genetic sequences are immobilized on the gold electrodes.

The detecting apparatus may be constituted, wherein the internal space part, the gold electrodes and the common electrode are contained in the detecting chip, and the detecting chip is inserted detachably and attachably into the measuring apparatus to be able to electrically connected to the measuring apparatus.

The detecting apparatus may be constituted, wherein temperature of the detecting chip is changed by using peltier devices to control temperature condition of hybridization.

The present invention, in order to solve the above problems, provides a method for detecting single base substitution SNP and point mutation of genes, the method comprising the steps of filling DNA samples or DNA amplified from DNA samples into the space part in the detecting apparatus to form a double strand by hybridization, filling an electrolyte including an electrochemically active molecule into the space part and controlling the temperature to bind the electrochemically active molecule with the double strand nucleic acid, and detecting single base substitution SNP and point mutation of DNA samples by detecting a flowing current value through the application of the voltage between the common electrode and the gold electrodes.

### Brief Description of the Drawings

Fig. 1 is a prospective view showing a whole structure of an example of the detecting apparatus for single base substitution SNP and point mutation of genes related to the present invention;
Fig. 2 is a prospective view explaining the whole structure of the example of the detecting chip employed for detecting single base substitution SNP and point mutation of genes related to the present invention;
Fig. 3 is a prospective view showing and explaining the conditions of use of the detecting chip;
Fig. 4 is a drawing showing the structure of the electrode and wiring, etc., of the detecting apparatus and the detecting chip of single base substitution SNP and point mutation of genes related to the present invention.

### Best Mode for carrying out the Invention

In order to explain the present invention more specifically, examples of application of the invention with reference to the attached drawings are described below.

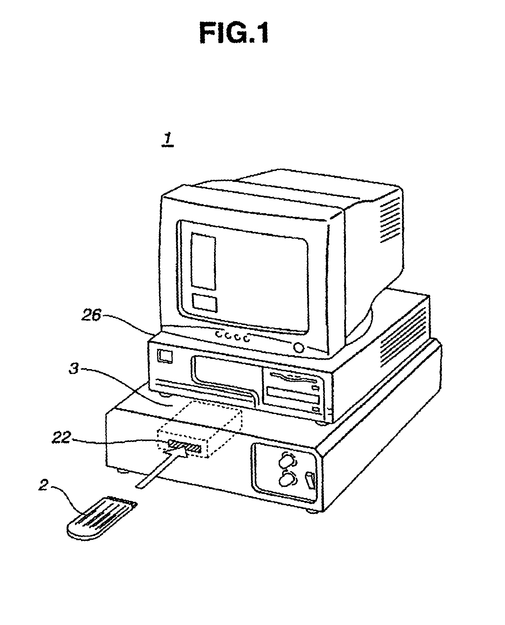

Fig. 1 illustrates an example of a detecting method for, and a detecting apparatus and a detecting chip of single base substitution SNP and point mutation of genes related to the present invention, in which a detecting apparatus 1 for single base substitution SNP and point mutation of genes in connection with the present invention consists of a detecting chip 2 for hybridization and a measuring apparatus 3 capable of detecting and analyzing the double-stranded DNA originated from hybridization by inserting the detecting chip 2.

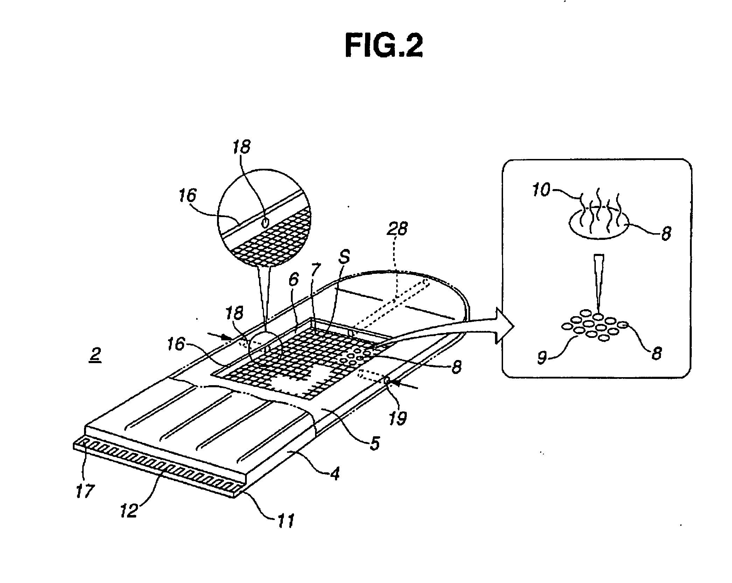

Referring to Fig. 2, the detecting chip 2 is made of ceramics or synthetic resin materials in the form of a card or a cassette, and consists of a body 4 and an upper cover 5 to be fixed to the body 4 from above (indicated by a imaginary line in the drawing). The detecting chip 2 is made from materials having tolerance to acid and alkali, and in particular, preferably the upper cover 5 is transparent for viewing the inside of the detecting chip 2 from the outside.

In the approximate center of the body 4, a rectangular depression 6 is formed. Thereby, when the body 4 is integrated with the upper cover 5 by fixing the upper cover 5, the depression part becomes a closed space part S. On the bottom of the space part S, that is, on the base 7 of the depression 6, gold spots are deposited in the form of a matrix, that correspond to the plurality of gold electrodes 8 formed in the shape of the gold electrodes array 9.

On the right side of Fig. 2, an enlarged view of the gold electrodes 8 is shown. As shown in the enlarged view, PCR products and SH-oligonucleotides 10, whose SH radical is introduced by thiolization at the 5' end of the oligonucleotides, are immobilized on the gold electrodes 8. The PCR products are double-stranded DNAS, but the single strand oligonucleotides, thiolized by the introduction of SH radicals at the 5' end are 20-50 base long and immobilized on the gold electrodes 8 through thiole radicals introduced into the 5' end.

In the point 11 of the detecting chip 2, a plurality of terminals 12 are established side by side. Each of the gold electrodes 8 is respectively combined with each of the wirings 13, and the other ends of the wirings are spread to be respectively combined with the terminals 12.

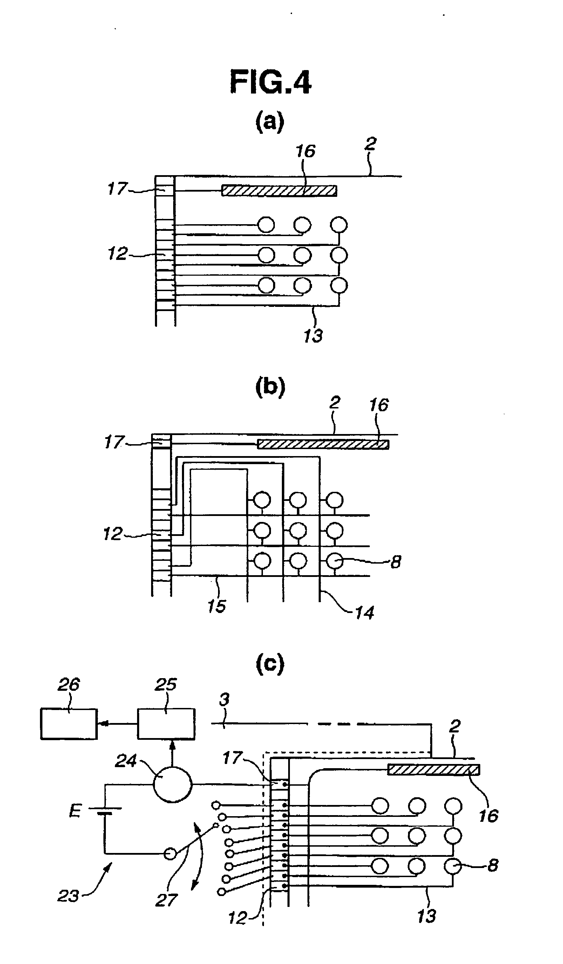

Moreover, as shown in Fig. 4 (a), each of the wirings for the gold electrodes 9 may be respectively connected with each of the gold electrodes 8 to connect with each of the terminals 12. However, as shown in Fig. 4 (b), the matrix wiring structure is formed as a cancellous wiring consisting of a plurality of conductors 14 and the conductor 15 fixed in rows and lines, which are utilized in the liquid crystal display to connect each of the gold electrodes 9 arranged in the form of the array with each of the nearest conductors fixed in rows and lines.

In this example, a common electrode 16 which is a counter electrode to the gold electrodes 9 is arranged at the position not contacting the gold electrodes 9 arranged in the form of the matrix at the bottom of the depression 6. The wiring of the common electrode 16 is formed by the deposition of gold as in the gold electrodes. The common electrode 16 is spread to be connected with a terminal 17 for the common electrode. Furthermore, the electric current value between each of the gold electrodes 9 and the common electrode 16, which is a counter electrode to the gold electrodes 9, is measured on the basis of the value of a reference electrode 28 that is wired to contact the space within the depression 6, and a structure capable of obtaining the correct current value of every measure is formed.

In both sides of the body 4 and the upper cover 5 of the detecting chip 2, injection holes 18 and 19 (reference to the enlarged view of a substantial part of the injection hole indicated at the top of Fig. 2) are formed for communicating to the depression 6, and they are usually closed by cap plugs so that the closed space s is formed. By removing the caps, disposal injectors 20 and 21 may be inserted into the injection holes 18 and 19. Thereby, solution may be injected into the closed space part S generated by the depression 6 and the upper cover 5 or the exchange and mixture of the solution within the space part S may be promptly conducted.

A measuring apparatus 3 comprises an insertion slot 22 for inserting the deducting chip 2. Within the insertion slot 22, a circuit 23 is established as shown in Fig. 4 (c), for connecting to the terminal 17 for the common electrode and the terminal 12 for each of the gold electrodes and applying voltage between the terminal 17 for the common electrode and the terminal 12 for each of the gold electrodes. The measuring apparatus 3 is constructed to detect and measure the electric current flowing between the common electrode 16 and each of the gold electrodes 8 in the detector 24 established in the circuit 23, upon the application of voltage between the terminal 17 for the common electrode and the terminal 12 for each of the gold electrodes.

The measurement data based on the deducted electric current is digitized by an A-D converter 25, etc., connected to the detector 24, and is utilized as processing data of analysis or identification of samples in a personal computer 26. Moreover, a temperature control apparatus that consists of peltier devices is equipped in the measuring apparatus 3.

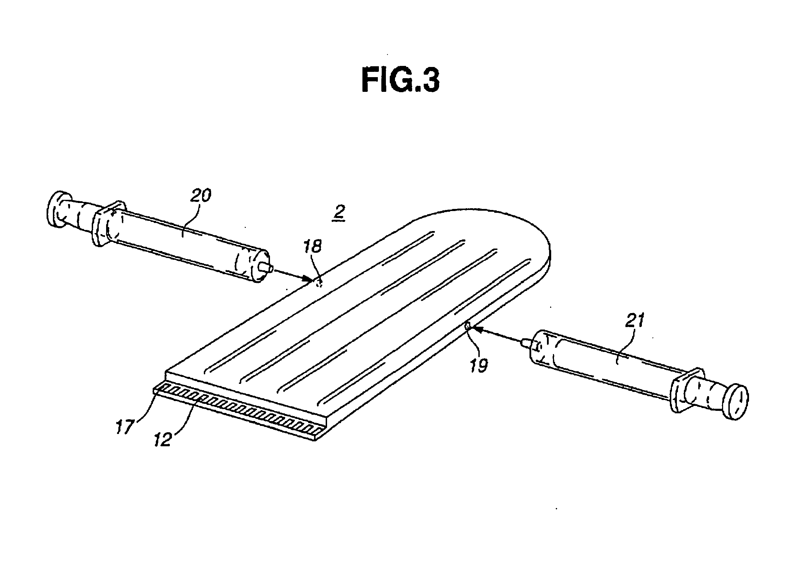

Hereafter, the detecting apparatus 1 for single base substitution SNP and point mutation of genes related to the present invention constituted as prescribed above is described. The detecting chip 2 is sealed between the body 4 and the upper cover which are combined. As shown Fig. 3, the injector 20 and 21 are inserted in the injection hole 18 and 19 to inject solution containing DNA samples.

Furthermore, as DNA samples, DNAs which are extracted from biomaterials and then cleaved by DNA lyase or by the supersonic treatment or DNAS from specific genes which are amplified by PCR (polymerase chain reaction) are used. The DNA samples are denatured by a heat treatment immediately before hybridization.

When DNA samples (single-stranded) are mixed to the immobilized PCR products or oligonucleotides (single-stranded), the hybridization is conducted between the PCR products or oligonucleotides and DNA samples that have complementary base sequences to each other. At this moment, the detecting chip 2 is fixed by inserting it into the insertion slot 22 in the measuring apparatus to control the temperature by using peltier devices furnished in the measuring apparatus 3, so that the temperature condition during hybridization is controlled.

After the hybridization, the detecting chip 2 is pulled out of the measuring apparatus 3, and DNA samples which did not hybridize are washed by injecting a washing solution from the injection hole 18 by the injector and then absorbing the solution within space part S from the other injection hole 19.

After washing, electrolytic solution including electrochemically active molecules are injected into the space part S from the injection hole 18 and 19 by the injector. The electrochemically active molecules perform the function of changing electric characteristics such as the value of the resistance of double-stranded DNA by hybridization. This point is specifically described in the official gazette, Patent Laid-Open Publication Hei 9(1997)-288080.

When the detecting chip 2 after the treatment described above is refixed to the measuring apparatus 3, and the terminal 17 for the common electrode and the terminal 12 for each of the gold electrodes are connected to the voltage circuit 23, and thereafter, weak voltage is applied between the common electrode 16 and each of the gold electrodes 8, a weak electric current flows between the double-stranded DNA generated by hybridization and the connected gold electrodes 8 through the voltage circuit 23 and the common electrode 16. Temperature is controlled by peltier devices furnished within the measuring apparatus 3 and current values in different temperatures are measured.

In the measuring apparatus 3, as shown in Fig. 4 (c), the electric current sequentially flows through the double-stranded DNA after hybridization, and detection is conducted by automatically switching a scanning terminal 27 to each of the terminals of gold electrodes 12. The detection results are converted into digital data by the A-D converter, etc., and accumulated into the memory of the personal computer as measurement data. By the measurement data, the identification and the analysis of DNA samples are conducted. For example, by comparing the measurement data to each kind of DNA data previously accumulated, the identification or the analysis of DNA samples may be conducted.

Next, experimental examples of the electrochemic detecting apparatus for a base substitution and point mutation, etc., of genes in accordance with the present invention are described.

Firstly, experimental example 1 is described.

An experimental example of the detection of single base substitution SNP in the seventy second codon of gene p53 is stated. Each of the oligonucleotides comprising base sequences corresponding to the following two kinds of polymorphisms (genetic polymorphism) is immobilized on each of the gold electrodes by spotting.
P53Pro (the seventy second codon is Pro)
P53Arg (the seventy second codon is Arg)

DNA obtained from peripheral blood of a normal person who has Pro in the seventy second codon of p53 and the PCR products whose area including codon 72, that exists in exon 4 of p53, is amplified from such DNA are denatured, and thereafter, hybridization reaction is conducted. In 0.1M AcOH-AcOK (pH5.6), 0.1M KC1 and 0.05mM Nfc as measuring electrolytic solution, variations in current value before and after hybridization at 470 mV, 20°C (Ag/AgCl reference electrode standard), are measured.

| DNA obtained from peripheral blood | |
|---|---|
| Current variation of p53Pro (%) | 52% |
| Current variation of p53Arg (%) | 15% |

| PCR products | |
|---|---|
| Current variation of p53Pro (%) | 65% |
| Current variation of p53Arg (%) | 13% |

Furthermore, similarly, hybridization reaction is conducted to DNA obtained from peripheral blood of a normal person whose seventy second codon of p53 is Arg and the PCR products whose area including codon 72 that exists in exon 4 of p53 is amplified from such DNA.

| DNA obtained from peripheral blood | |
|---|---|
| current variation of p53Pro (%) | 46% |
| Current variation of p53Arg (%) | 17% |

| PCR products | |
|---|---|
| Current variation of p53Pro (%) | 53% |
| Current variation of p53Arg (%) | 11% |

It is clear that these current variations are different depending on if the base sequence matches completely or if there is a mismatch.

Next, experimental example 2 is described.

The experimental example illustrating that the measured current value depends on the number of base sequences, and thereby, the number of mismatched base sequences can be measured is prescribed. Seven kinds of oligonucleotides, dT20, dT10dAdT9, dT8dA4dT8, dAdT19, dA3dT17, dT19dA and dT17dA3 are immobilized to each of the gold electrodes by spotting. Hybridization of dA20 is conducted to each of the oligonucleotides.

In 0.1M AcOH-AcOK (pH 5.6), 0.1M KC1 and 0.05mM NFc as measuring electrolytic solution, variations in current value before and after hybridization in 470mV (Ag/AgCl reference electrode standard) at 20°C are measured. The measurement results are shown in Table 1.

**(Table 1)**

| | dT20 | dT10d AdT9 | dT8dA 4dT8 | dAd T19 | DA3dT 17 | dT19 dA | dT17d A3 |
|---|---|---|---|---|---|---|---|
| Current variation(%) | 37 | 22 | 15 | 14 | 14 | 20 | 12 |
| Tm (°C) | 46 | 36 | 21 | 45 | 46 | 42 | 41 |

The current variations shown in Table 1 depend on the number of mismatched bases. Particularly, when the terminal parts of the sequence are mismatched, current variations greater than that of the Tm value were observed. In the conventional SSCP, it was impossible to detect the above results, but the present invention clarified such relationship.

### Industrial Applicability

A method for detecting single base substitution SNP and point mutation of genes and a detecting apparatus and a detecting chip for single base substitution SNP and point mutation of genes in connection with the present invention is constructed as mentioned above, so that it becomes possible to detect and analyze a large number of single base substitution SNP and point mutation for a plurality of DNA samples with high sensitivity.

Accordingly, the detecting apparatus capable of conducting the treatment with high sensitivity and high-throughput in connection with the present invention is an effective means for analyzing the correlation between genes and phenotypes in the biological or medical science field. Additionally, the detecting or analyzing apparatus for specific base sequence SNP and point mutation in connection with the present invention may be utilized in the field of gene diagnosis by analyzing specific genes such as drug metabolic enzymes or cancer repressor genes.

For example, the detecting apparatus in connection with the present invention is capable of conducting the analysis with high sensitivity and high-throughput, so that it may collect the data of single base substitution SNP and point mutation of Japanese people, identify the single base substitution SNP and point mutation related to diseases in order to prevent cancer or other geriatric diseases such as hypertension.

## Claims

1. A method for detecting single base substitution SNP and point mutation of genes using a detecting apparatus (1) wherein the detecting apparatus (1) comprises:
a detecting chip (2), and
a measuring apparatus (3) capable of detecting electric current through the application of voltage between a common electrode (16) and gold electrodes (8)
wherein the detecting chip (2) comprises:
a closed internal space part (S) capable of being filled with DNA samples and allowing to remove DNA samples,
a plurality of gold electrodes (8) corresponding to measuring electrodes formed at the bottom of said space part (S), and
a common electrode (16) which is a counter electrode to said gold electrodes (8), said common electrode being arranged not to contact said gold electrodes in said space part (S),
wherein PCR products or oligonucleotides(10) consisting of different genetic sequences are immobilized on each of said gold electrodes (8), and
wherein voltage is applied between said common electrode (16) and said gold electrodes (8) so that electric current which is formed between said common electrode and said gold electrodes could be detected,
said method comprising the steps of:
filling DNA samples or gene-amplified DNAs from DNA samples into the space part (S) in the detecting apparatus (1) to form a double strand by hybridization with the PCR products or the oligonucleotides;
filling an electrolyte including electrochemically active molecules into said space part, and controlling the temperature such that the electrochemically active molecules bind with said double strand; and
detecting single base substitution SNP and point mutation of DNA samples by detecting the value of a current flowing upon application of a voltage between said common electrode (16) and said gold electrodes (8).

2. The method according to claim 1,
wherein a plurality of said gold electrodes (8) are respectively connected with wirings (13), and
each of said wirings (13) is respectively connected with a terminal, or
said wirings form a matrix structure to connect each of said gold electrodes (8) arranged in the form of an array with the nearest conductor fixed in rows (14) or lines (15) as cancellous wirings consisting of a plurality of conductors fixed in rows or lines.

3. The method according to claim 1 or claim 2, wherein the detecting chip (2) is constituted to be able to be fixed to the measuring apparatus (3) capable of detecting electric current detachably and attachably, and to be able to electrically connect to the measuring apparatus (3).

4. The method according to any one of the claims 1 to 3, wherein the detecting chip (2) is a chip in the form of a card or a cassette.

5. The method according to any one of the claims 1 to 4, wherein the detecting chip consists of a body (4) and an upper cover (5) to fit to said body (4) from upward.

6. A method according to claim 1,
wherein said detecting chip (2) is inserted detachably and attachably into said measuring apparatus (3) and is able to be electrically connected to said measuring apparatus.

7. A method according to claim 6,
wherein the temperature of said detecting chip (2) is controlled by using peltier devices.

## Patentansprüche

1. Verfahren zum Erfassen von Einzelbasensubstitutions-SNP und Punktmutation von Genen unter Verwendung einer Erfassungsvorrichtung (1), wobei die Erfassungsvorrichtung (1) umfasst:
einen Erfassungs-Chip (2), und
eine Messvorrichtung (3), die in der Lage ist, elektrischen Strom über das Anlegen einer Spannung zwischen einer gemeinsamen Elektrode (16) und Goldelektroden (8) zu erfassen,
wobei der Erfassungs-Chip (2) umfasst:
einen geschlossenen Innenraumteil (S), der mit DNA-Proben gefüllt werden kann und das Entfernen von DNA-Proben zulässt,
eine Vielzahl von Goldelektroden (8), die Messelektroden entsprechen, die am Boden des Raumteils (S) ausgebildet sind, und
eine gemeinsame Elektrode (16), die eine Gegenelektrode zu den Goldelektroden (8) ist, wobei die gemeinsame Elektrode so angeordnet ist, dass sie nicht mit den Goldelektroden in dem Raumteil (S) in Kontakt kommt,
wobei PCR-Produkte oder Oligonukleotide (10), die aus unterschiedlichen genetischen Sequenzen bestehen, an jeder der Goldelektroden (8) immobilisiert werden, und wobei Spannung zwischen der gemeinsamen Elektrode (16) und den Goldelektroden (8) angelegt wird, so dass elektrischer Strom, der zwischen der gemeinsamen Elektrode und den Goldelektroden erzeugt wird, erfasst werden könnte,
wobei das Verfahren die folgenden Schritte umfasst:
Füllen von DNA-Proben oder genamplifizierten DNA aus DNA-Proben in den Raumteil (S) in der Erfassungsvorrichtung (1), um durch Hybridisierung mit den PCR-Produkten oder den Oligonukleotiden einen Doppelstrang auszubilden;
Füllen eines Elektrolyts, der elektrochemisch aktive Module enthält, in den Raumteil und Steuern der Temperatur so, dass die elektrochemisch aktiven Moleküle Bindung mit dem Doppelstrang eingehen; und
Erfassen von Einzelbasensubstitutions-SNP und Punktmutation von DNA-Proben durch Erfassen des Wertes eines Stroms, der beim Anlegen einer Spannung zwischen der gemeinsamen Elektrode (16) und den Goldelektroden (8) fließt.

2. Verfahren nach Anspruch 1,
wobei eine Vielzahl der Goldelektroden (8) jeweils mit Verdrahtungen (13) verbunden sind, und
jede der Verdrahtungen (13) jeweils mit einem Anschluss verbunden ist, oder die Verdrahtungen eine Matrixstruktur zum Verbinden jeder der Goldelektroden (8), die in Form eines Feldes angeordnet sind, mit dem nächstgelegenen Leiter, fixiert in Reihen (14) oder Zeilen (15), als gitterförmige Verdrahtungen bilden, die aus einer Vielzahl von Leitern bestehen, die in Reihen oder Zeilen fixiert sind.

3. Verfahren nach Anspruch 1 oder Anspruch 2, wobei der Erfassungs-Chip (2) so aufgebaut ist, dass er an der Messvorrichtung (3), die in der Lage ist, elektrischen Strom zu erfassen, abnehmbar und anbringbar befestigt werden kann, und so, dass er elektrisch mit der Messvorrichtung (3) verbunden werden kann.

4. Verfahren nach einem der Ansprüche 1 bis 3, wobei der Erfassungs-Chip (2) ein Chip in Form einer Karte oder einer Kassette ist.

5. Verfahren nach einem der Ansprüche 1 bis 4, wobei der Erfassungs-Chip aus einem Körper (4) und einer oberen Abdeckung (5) besteht, die von oben auf den Körper (4) passt.

6. Verfahren nach Anspruch 1,
wobei der Erfassungs-Chip (2) abnehmbar und anbringbar in die Messvorrichtung (3) eingeführt wird und elektrisch mit der Messvorrichtung verbunden werden kann.

7. Verfahren nach Anspruch 6,
wobei die Temperatur des Erfassungs-Chips (2) unter Verwendung von Peltier-Einrichtungen gesteuert wird.

## Revendications

1. Procédé pour détecter des SNP à substitution d'une seule base et des mutations ponctuelles de gènes au moyen d'un appareil de détection (1) où l'appareil de détection (1) comprend :
une puce de détection (2) et
un appareil de mesure (3) capable de détecter un courant électrique par application d'une tension entre une électrode commune (16) et des électrodes en or (8),
où la puce de détection (2) comprend :
une partie formant espace interne fermé (S) capable d'être remplie avec des échantillons d'ADN et permettant de retirer des échantillons d'ADN,
une pluralité d'électrodes en or (8) correspondant à des électrodes de mesure formées au fond de ladite partie formant espace (S), et
une électrode commune (16) qui est une contre-électrode pour lesdites électrodes en or (8), ladite électrode commune étant disposée pour ne pas être en contact avec lesdites électrodes en or dans ladite partie formant espace (S),
où des produits de PCR ou des oligonucléotides (10) consistant en différentes séquences génétiques sont immobilisés sur chacune desdites électrodes en or (8), et
où une tension est appliquée entre ladite électrode commune (16) et lesdites électrodes en or (8) de sorte que le courant électrique qui est formé entre ladite électrode commune et lesdites électrodes en or peut être détecté,
ledit procédé comprenant les étapes de :
introduire des échantillons d'ADN ou des ADN de gènes amplifiés provenant d'échantillons d'ADN dans la partie formant espace (S) dans l'appareil de détection (1) pour former un double brin par hybridation avec les produits de PCR ou les oligonucléotides ;
introduire un électrolyte incluant des molécules électrochimiquement actives dans ladite partie formant espace et réguler la température de sorte que les molécules électrochimiquement actives se lient audit double brin ; et
détecter les SNP à substitution d'une seule base et les mutations ponctuelles d'échantillons d'ADN en détectant la valeur d'un courant circulant lors de l'application d'une tension entre ladite électrode commune (16) et lesdites électrodes en or (8).

2. Procédé selon la revendication 1
où une pluralité desdites électrodes en or (8) sont connectées respectivement avec des câblages (13) et
chacun desdits câblages (13) est connecté respectivement à une borne, ou
lesdits câblages forment une structure de matrice pour connecter chacune desdites électrodes en or (8) disposées sous forme d'une matrice avec le conducteur le plus proche fixé en colonnes (14) ou en lignes (15) sous forme de câblages réticulés consistant en une pluralité de conducteurs fixés en colonnes ou en lignes.

3. Procédé selon la revendication 1 ou la revendication 2 où la puce de détection (2) est constituée pour être capable d'être fixée à l'appareil de mesure (3) capable de détecter un courant électrique de manière détachable et attachable, et pour être capable d'être connectée électriquement à l'appareil de mesure (3).

4. Procédé selon l'une quelconque des revendications 1 à 3 où la puce de détection (2) est une puce sous forme d'une carte ou d'une cassette.

5. Procédé selon l'une quelconque des revendications 1 à 4 où la puce de détection consiste en un corps (4) et en un élément de recouvrement supérieur (5) destiné à s'ajuster audit corps (4) par le haut.

6. Procédé selon la revendication 1
où ladite puce de détection (2) est insérée de manière détachable et attachable dans ledit appareil de mesure (3) et est capable d'être connectée électriquement audit appareil de mesure.

7. Procédé selon la revendication 6 où la température de ladite puce de détection (2) est régulée au moyen de dispositifs peltier.
